# EUROPÄISCHE PATENTSCHRIFT

(11) **EP 2 564 763 B1**
(45) Veröffentlichungstag und Bekanntmachung des Hinweises auf die Patenterteilung: **11.10.2017**
(21) Anmeldenummer: 12180842.2
(22) Anmeldetag: 17.08.2012
(51) Int. Cl.: A61B 3/10, A61B 3/107

(54) **Ophthalmologisches Analysegerät und Verfahren**
Ophthalmological analysis apparatus and method
Appareil d'analyse ophtalmologique et procédé

(30) Priorität: 30.08.2011 DE 102011081825
(43) Veröffentlichungstag der Anmeldung: 06.03.2013
(73) Patentinhaber: Oculus Optikgeräte GmbH, 35582 Wetzlar-Dutenhofen (DE)
(72) Erfinder: Steinmüller, Andreas, 35435 Wettenberg (DE)
(74) Vertreter: advotec.

(56) Entgegenhaltungen:
- EP-A1- 2 016 888
- WO-A1-2011/093209
- US-A1- 2006 263 297
- US-A1- 2009 168 019

## Beschreibung

Die Erfindung betrifft ein ophthalmologisches Analysegerät mit den Merkmalen des Oberbegriffs des Anspruchs 1.

Bei den aus dem Stand der Technik bekannten Analysegeräten bzw. Topografiesystemen wird regelmäßig ein Auge mit monochromatischen, wie beispielsweise infrarotem Licht beleuchtet, um eine Blendung der zu untersuchenden Person bzw. des Probanden weitestgehend zu vermeiden. So sind Topografiesysteme bekannt, die neben einer Messung einer Oberfläche eines Auges, auch pupillometrische Messungen ermöglichen. Insbesondere bei einer Beleuchtung des Auges mit monochromatischen, nur bedingt sichtbaren Licht wird eine Kontraktion der Pupille wirkungsvoll vermieden, sodass sich eine derartige Beleuchtung besonders gut für pupillometrische Messungen eignet. Topografiesysteme können aber auch über mehrere Lichtfarben zur Beleuchtung eines Auges verfügen. Dann werden zum Beispiel Placidoringe in unterschiedlichen, monochromatischen Lichtfarben auf das Auge projiziert. Dies dient im Wesentlichen zur Unterscheidung der Ringe des auf dem Auge abgebildeten Bildmusters und alleine zum Zwecke einer Topographiebestimmung. Derartige Placidoringe in jeweils unterschiedlichen, monochromatischen Lichtfarben bilden folglich alleine die erste Lichtquelle aus. Weiter ist es bekannt, eine Untersuchung von Meibomdrüsen unter infrarotem Licht durchzuführen.

Auch können Analysegeräte zur Messung einer Topografie bzw. sogenannte Keratometer zur nicht invasiven Analyse eines Tränenfilms auf einem Auge verwendet werden. Dabei wird das auf die Oberfläche des Auges projizierte Bildmuster im Wesentlichen kontinuierlich aufgenommen, wobei ein Aufreißen des Tränenfilms durch eine Veränderung des Bildmusters erkannt werden kann. Um eine Aussage über eine Qualität des Tränenfilms treffen zu können, wird regelmäßig so eine Aufreißzeit desselben gemessen. Diese Messung wird ebenfalls mittels einer Beleuchtung des Auges mit infrarotem Licht durchgeführt. Beispielsweise ist eine Messung eines Tränenfilms, neben der Topografie der Oberfläche des Auges, bei einer Auswahl von Kontaktlinsen von großer Bedeutung. Weiter ist eine Analyse eines Tränenfilms alleine auf eine Verteilung des Tränenfilms über das Auge beschränkt. Nachteilig bei dem bekannten Analysegerät bzw. Verfahren ist, dass die Möglichkeiten zur Untersuchung eines Auges begrenzt sind. Es ist daher wünschenswert, die Untersuchungsmöglichkeiten eines derartigen Gerätes zu erweitern, um gegebenenfalls weitere und detaillierte Messergebnisse zu erhalten, die beispielsweise für eine verbesserte Kontaktlinsenauswahl und Anpassung nutzbar sind.

Die EP 2 016 888 A1 beschreibt ein ophthalmologisches Analysegerät zur Messung einer Topographie einer Oberfläche eines Auges. Das Analysegerät umfasst eine Projektionsvorrichtung mit einer Beleuchtungseinrichtung und einer Blendeneinrichtung. Die Blendeneinrichtung ist dabei von einer Placidoscheibe ausgebildet. Weiter weist die Beleuchtungseinrichtung eine erste Lichtquelle auf, welche rückwärtig der Placidoscheibe angeordnet ist und aus roten Licht emittierenden Leuchtdioden gebildet ist. So ist ein Bildmuster auf einer Oberfläche eines Auges abbildbar, wobei mittels einer Beobachtungsvorrichtung Bilder des abgebildeten Bildmusters aufgenommen werden können und wobei aus den Bildern eine Topographie der Oberfläche abgeleitet werden kann. Es kann eine weitere Lichtquelle vorgesehen sein, die ebenfalls rückwärtig der Placidoscheibe angeordnet und aus Leuchtdioden gebildet ist, die normales Licht emittieren können.

Die US 2009/0168019 A1 beschreibt ebenfalls ein ophthalmologisches Analysegerät mit einer Placidoscheibe und einer Beleuchtungseinrichtung. Die Placidoscheibe verfügt über eine Beleuchtungseinrichtung mit einer ersten Lichtquelle, wobei ein Auge mit Licht von der Placidoscheibe so beleuchtet wird, dass mittels einer Kamera Bilder aufgenommen werden können, die eine Ableitung einer Topographie der Oberfläche des Auges ermöglichen. Die rückseitig der Placidoscheibe angeordnete erste Lichtquelle ist nicht näher offenbart. Eine weitere Lichtquelle bzw. Leuchtdiode des Geräts dient zur unmittelbaren Projektion von infrarotem Licht auf die Oberfläche des Auges. Eine Bestimmung eines Tränenfilms bzw. eines Tränenvolumens erfolgt insbesondere durch eine Messung einer Höhe eines Meniskus.

Die US 2006/0263297 A1 offenbart ein ophthalmologisches Analysegerät, mittels dem ein Tränenfilmmeniskus fokussierbar ist. Einer Tränenflüssigkeit wird eine fluoreszierende Substanz zugesetzt, wobei in einem Strahlengang einer Beleuchtungseinrichtung ein Filter eingeschoben wird. Dieser Filter dient zur Erzeugung eines Lichts mit einem Spektrum, welches die fluoreszierende Substanz anregen kann. So wird die Oberfläche des Auges bzw. der Tränenfilmmeniskus mit monochromatischem Licht beleuchtet.

Die WO 2011/093209 A1 beschreibt ein Analysegerät zur Bestimmung eines Tränenfilms. Ein Auge wird hier mit weißem Licht beleuchtet und ein Reflektionsbild mit einer Farbkamera aufgenommen. Weiter ist es mit dem Gerät möglich eine Lipidschicht zu bestimmen.

Der vorliegenden Erfindung liegt daher die Aufgabe zu Grunde, ein ophthalmologisches Analysegerät vorzuschlagen, mit dem die Untersuchungsmöglichkeiten eines Topografiesystems mit einfachen Mitteln erweitert und verbessert werden.

Diese Aufgabe wird durch eine Vorrichtung mit den Merkmalen des Anspruchs 1 gelöst.

Das erfindungsgemäße, ophthalmologische Analysegerät, insbesondere zur Messung einer Topografie einer Oberfläche eines Auges, weist eine Projektionsvorrichtung und eine Beobachtungsvorrichtung auf, wobei die Projektionsvorrichtung zumindest eine Beleuchtungseinrichtung und eine Rasterblende umfasst, wobei die Rasterblende aus einem transparenten Korpus mit kreisringförmigen Blendenelementen gebildet ist, die auftreffendes Licht aus einer Lichtquelle in einen Blendenraum reflektieren, wobei die Beleuchtungseinrichtung zumindest eine erste Lichtquelle aufweist, die Licht in einem monochromatischen Spektrum emittieren kann, wobei auf die Rasterblende Licht der ersten Lichtquelle auftrifft, derart, dass mittels der Rasterblende ein Bildmuster mit monochromatischen Licht der ersten Lichtquelle auf einer Oberfläche eines Auges abbildbar ist, wobei mittels der Beobachtungsvorrichtung Bilder des abgebildeten Bildmusters aufgenommen werden können, wobei aus den Bildern eine Topografie der Oberfläche abgeleitet werden kann, wobei die Beleuchtungseinrichtung zumindest eine weitere Lichtquelle aufweist, die polychromatisches Licht in einem sichtbaren Spektrum zur Beleuchtung emittieren kann, wobei auf die Rasterblende Licht der weiteren Lichtquelle auftrifft, derart, dass mittels der Rasterblende ein Bildmuster mit sichtbaren polychromatischen Licht der weiteren Lichtquelle auf der Oberfläche eines Auges abbildbar ist, wobei mittels der Beobachtungsvorrichtung Bilder des abgebildeten Bildmusters aufgenommen werden können.

Neben der Verwendung von im wesentlichen monochromatischen Licht bzw. Licht eines vergleichsweise schmalen Wellenlängenbereichs ermöglicht die weitere Lichtquelle eine Beleuchtung der Oberfläche des Auges mit polychromatischen, sichtbaren Licht, wodurch zusätzliche Messungen von Eigenschaften des Auges und eine Erzielung genauerer Messergebnisse möglich werden. Gegenüber monochromatischen oder infraroten Licht wird es möglich mit polychromatischen Licht einen Rötungsgrad des Auges zu bestimmen. Auch wird es möglich, eine Dicke eines Tränenfilms oder einer Lipidschicht des Tränenfilms vergleichsweise genau nicht invasiv zu messen, da mit polychromatischen Licht farbige Interferenzmuster der Lipidschicht erzeugt werden können, die für derartige Messungen verwendbar sind.

Vorteilhaft kann die erste Lichtquelle Licht in einem überwiegend infrarotem Spektrum emittieren. Infrarotes Licht hat eine besonders geringe Blendwirkung auf das zu untersuchende Auge und ist einfach zur Verfügung zu stellen.

Besonders vorteilhaft ist es, wenn die weitere Lichtquelle überwiegend weißes Licht emittieren kann. Mit weißem Licht kann eine besonders gute Farbwiedergabe erzielt werden, was insbesondere bei einer Messung des Rötungsgrades des Auges und der Erzeugung farbiger Interferenzmuster eine Messgenauigkeit wesentlich verbessert.

In einer Ausführungsform kann die weitere Lichtquelle aus einer Vielzahl gleichmäßig verteilter Leuchtdioden zusammengesetzt sein. Beispielsweise können die Leuchtdioden selbst das Bildmuster ausbilden. So können die Leuchtdioden ringförmig angeordnet sein oder auch hinter einer Blende, die das Bildmuster auf das Auge projizieren kann. Die Leuchtdioden, die die weitere Lichtquelle ausbilden, können dabei zusammen mit Leuchtdioden, die die erste Lichtquelle ausbilden, an der Beleuchtungseinrichtung angeordnet sein. So wird es möglich, die betreffenden Leuchtdioden nach Bedarf, getrennt voneinander oder zusammen zu betreiben. Eine Anzahl der Leuchtdioden der ersten Lichtquelle kann dabei zu einer Anzahl der Leuchtdioden der weiteren Lichtquelle in einem Verhältnis von 1 zu 3 gewählt sein.

Um qualitativ gut verwertbare Bilder zu erhalten, kann die Beobachtungsvorrichtung in einer nicht der Erfindung zughörigen Ausführungsform eine Kamera und ein Objektiv aufweisen, wobei mittels des Objektivs eine Vergrößerung des Bildes veränderbar sein kann. So wird es möglich, je nach Art der Messung eine Vergrößerung des Objektivs so zu wählen, dass das zu messende Objekt bzw. die zu messende Eigenschaft so visuell darstellbar ist, dass überhaupt eine Auswertung des Bildes sowie besonders genaue Messergebnisse möglich werden.

Das Objektiv kann dazu in einer nicht der Erfindung zughörigen Ausführungsform einen Vergrößerungswechsler aufweisen, mittels dem zumindest eine Linse in einen Strahlengang des Objektivs einbringbar bzw. aus diesem entfernbar ist. Gegenüber einer variablen Vergrößerungseinstellung, welche ebenfalls denkbar ist, ist ein Vergrößerungswechsler besonders einfach und kostengünstig herstellbar. Auch wird es dann möglich immer gleichbleibende, standardisierte Vergrößerungen für die verschiedenen Messungen zu verwenden, was die Messungen sowie eine Auswertung bzw. Analyse der Bilder erheblich vereinfacht. Mittels des Objektivs können vorteilhaft zumindest drei Vergrößerungen ausbildbar sein. Eine erste, normale Vergrößerung kann zur Messung der Topografie der Oberfläche des Auges verwendet werden. Eine zweite, große Vergrößerung, kann zur Messung bzw. Analyse eines Tränenfilms verwendet werden. Dabei kann insbesondere bei geringer Tiefenschärfe beispielsweise auf eine Lipidschicht fokussiert werden, um deren Dicke zu bestimmen. Eine dritte, kleine Vergrößerung kann insbesondere für meibometrische Untersuchungen genutzt werden, da eine Bildaufnahme von Meibomdrüsen unter anderem einen vergrößerten Abstand des Analysegeräts relativ zum Auge erfordert.

Besonders vorteilhaft ist es in einer nicht der Erfindung zughörigen Ausführungsform, wenn in einer Richtung einer optischen Achse des Auges ausrichtbaren Geräteachse der Blendeneinrichtung ein Durchlass in der Blendeneinrichtung für einen Strahlengang der Beobachtungsvorrichtung ausgebildet ist. So kann die Oberfläche des Auges durch die Blendeneinrichtung allseitig mit dem Bildmuster beleuchtet werden. Die Blendeneinrichtung kann so beispielsweise aus einem leuchtenden Ring oder einer Mehrzahl derartiger, konzentrischer Ringe zur Erzeugung eines Placidobildmusters ausgebildet sein. Wenn die Blendeneinrichtung nach der optischen Achse des Auges mit ihrer Geräteachse ausgerichtet ist, kann der Strahlengang der Beobachtungsvorrichtung unmittelbar entlang der optischen Achse oder auf dieser verlaufen, sodass eine frontale Beobachtung des Auges durch den Durchlass hindurch ermöglicht und dann insbesondere pupillometrische Messungen wesentlich vereinfacht.

So kann in einer nicht der Erfindung zughörigen Ausführungsform das Analysegerät auch eine Blendvorrichtung zur Erzeugung eines Blendreizes auf das Auge aufweisen, wobei die Blendvorrichtung eine Blendlichtquelle und einen Strahlenteiler zur Einspiegelung der Blendlichtquelle in den Strahlengang der Beobachtungsvorrichtung aufweisen kann. So kann das Auge mit dieser Blendvorrichtung geblendet werden, wobei gleichzeitig eine Reaktion des Auges mittels der Beobachtungsvorrichtung aufgenommen werden kann. Zum Beispiel kann eine durch die Blendung veranlasste Bewegung der Pupille erfasst und ausgewertet werden. Die Blendvorrichtung kann dabei unabhängig von der Beleuchtungseinrichtung bzw. von mit dieser erzeugten Bildmustern betrieben werden.

Weiter ist es möglich, die Beleuchtungseinrichtung und/oder die Blendvorrichtung zur Blendung des Auges mit dem Ziel zu nutzen, dass eine vermehrte Produktion von Tränenflüssigkeit erfolgt. So kann die Bildung eines Tränenfilms überprüft bzw. gemessen werden.

Nachfolgend wird eine bevorzugte Ausführungsform der Erfindung unter Bezugnahme auf die beigefügten Zeichnungen näher erläutert.

Es zeigen:
- **Fig. 1:**: Eine vereinfachte, schematische Schnittansicht einer Ausführungsform eines Analysegerätes;
- **Fig. 2:**: eine Vorderansicht des Analysegerätes.

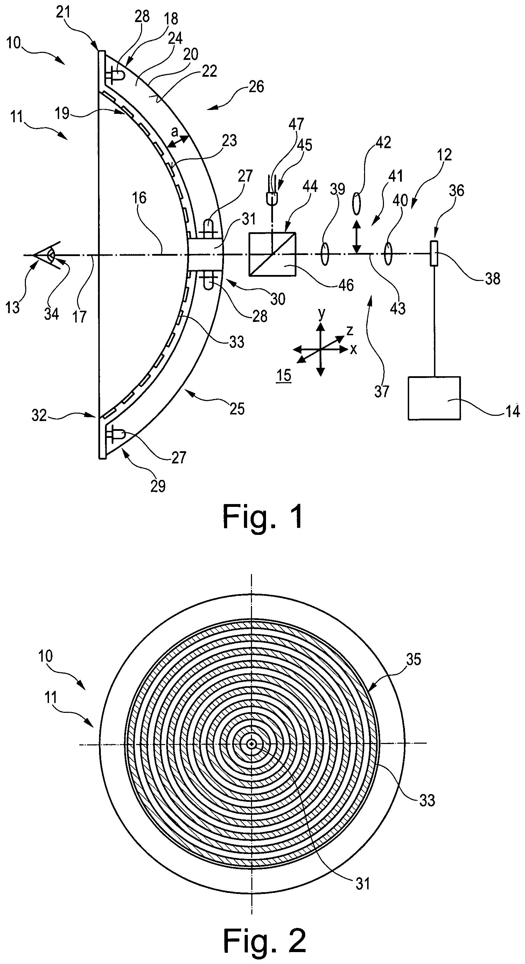

Eine Zusammenschau der **Fig. 1** und **2** zeigt eine Ausführungsform eines Analysegerätes 10, welches im Wesentlichen aus einer Projektionsvorrichtung 11 und einer Beobachtungsvorrichtung 12 zur Beobachtung eines Auges 13 gebildet ist. Weiter umfasst das Analysegerät 10 eine Auswertevorrichtung 14 mit hier nicht näher dargestellten Mitteln zur Datenverarbeitung und -ausgabe sowie eine Positioniervorrichtung 15 zur Positionierung des Analysegerätes 10 relativ zu dem Auge 13 in drei Raumrichtungen mit den senkrecht aufeinander stehenden Richtungskomponenten x, y, z, wie hier symbolisch angedeutet. Das Analysegerät wird dabei so positioniert, dass eine Geräteachse 16 des Analysegerätes 10 mit einer optischen Achse 17 des Auges 13 in Übereinstimmung gelangt.

Die Projektionsvorrichtung 11 ist als ein Hohlkugelsegment 18 ausgebildet und umfasst eine Rasterblende 19 und einen Reflektor 20, welche in einem schematisch angedeuteten Gehäuse 21 so angebracht sind, dass der Reflektor 20 mit einer reflektierenden Fläche 22 zu einer Oberfläche 23 des Reflektors 20 stets den gleichen Abstand a aufweist, und so ein gekrümmter Blendenraum 24 gebildet wird. Die Oberfläche 23 des Reflektors 20 ist stark reflektierend, so dass der Blendenraum 24 gleichmäßig lichterfüllt ist, wenn eine erste Lichtquelle 25 oder eine weitere Lichtquelle 26 eingeschaltet ist. Die Lichtquellen 25 und 26 sind jeweils aus Leuchtdioden 27 bzw. 28 gebildet, welche jeweils in einer gleichmäßig verteilten Vielzahl in Art eines Rings 29 an einem Umfang der Rasterblende 19 und in Art eines Rings 30 an einem Durchlass 31 der Rasterblende 19 angeordnet sind. Die Rasterblende 19 ist im Wesentlichen aus einem transparenten Korpus 32 mit kreisringförmigen Blendenelementen 33 gebildet, welche auftreffendes Licht aus einer Lichtquelle 25 und/oder 26 in den Blendenraum 24 reflektieren. Somit wird auf einer Hornhaut 34 des Auges 13 das in **Fig. 2** ersichtliche Bildmuster 35 abgebildet, wobei das Bildmuster 35 mittels der Beobachtungsvorrichtung 12 aufgenommen wird.

Die Beobachtungsvorrichtung 12 weist eine Kamera 36 mit einem Objektiv 37 auf, wobei die Kamera 36 unter anderem einen optischen Videosensor 38 umfasst, der unmittelbar mit der Auswertevorrichtung 14 verbunden ist. Das Objektiv 37 ist aus zwei Linsen 39 und 40 gebildet, wobei ergänzend, wie hier mit dem Doppelpfeil angedeutet, ein Vergrößerungswechsler 41 vorgesehen ist, mittels dem weitere Linsen 42 in einen Strahlengang 43 der Beobachtungsvorrichtung 12 eingeschwenkt werden können. Somit wird es möglich das Auge 13 in zumindest drei unterschiedlichen Vergrößerungen aufzunehmen und zu betrachten. In dem Strahlengang 43 ist zwischen der Projektionsvorrichtung 11 und dem Objektiv 37 ein Strahlenteiler 44 einer Blendvorrichtung 45 angeordnet, welcher aus einem Prismensystem 46 besteht, jedoch auch aus teildurchlässigen ebenen Spiegeln zusammengesetzt sein kann. Über dem Strahlenteiler 44 kann eine Blendlichtquelle 47 in dem Auge 13 abgebildet werden. Auf diese Weise kann man ganz unabhängig von der Projektionsvorrichtung 11 einen Blendreiz auf dem Auge 13 erzeugen, sodass dessen Reaktion mit Hilfe der ohnehin verfügbaren Beobachtungsvorrichtung 12 erfasst und von der Auswertevorrichtung 14 auch numerisch bestimmt werden kann. Ein spezielles Gerät ist für eine solche Messung dementsprechend nicht mehr erforderlich.

Hinsichtlich der Arbeitsweise des Analysegeräts 10 ist vorgesehen, dass die Leuchtdioden 27 Licht in einem überwiegend infraroten Spektrum und die Leuchtdioden 28 polychromatisches, weißes Licht in einem überwiegend sichtbaren Spektrum emittieren bzw. abstrahlen können. Die erste Lichtquelle 25 wird von circa 50 Leuchtdioden 27 und die weitere Lichtquelle 26 von circa 150 Leuchtdioden 28 gebildet. Innerhalb einer Messung kann nach Bedarf die erste Lichtquelle 25 oder die weitere Lichtquelle 26 zur Beleuchtung des Auges 13 eingeschaltet werden. Insbesondere für eine Bestimmung einer Topografie des Auges 13 ist es ausreichend, die erste Lichtquelle 25 bei einer normalen Vergrößerung des Objektivs 37 zu verwenden. Die erste Lichtquelle 25 wird auch für meibometrische Untersuchungen eingesetzt, wobei hier eine kleine Vergrößerung des Objektivs 37 gewählt wird und eine Vergrößerung eines Abstands des Analysegerätes 10 zu dem Auge 13 vorgesehen ist. Die weitere Lichtquelle 26 wird für eine Analyse eines Tränenfilms, insbesondere einer Lipidschicht des Tränenfilms eingesetzt, wobei dann eine besonders große Vergrößerung des Objektivs 37 gewählt wird. Im Übrigen wird die Blendlichtquelle 47 für pupillometrische Messungen eingesetzt.

## Patentansprüche

1. Ophthalmologisches Analysegerät (10), insbesondere zur Messung einer Topografie einer Oberfläche eines Auges, mit einer Projektionsvorrichtung (11) und einer Beobachtungsvorrichtung (12), wobei die Projektionsvorrichtung zumindest eine Beleuchtungseinrichtung und eine Rasterblende (19) umfasst, wobei die Rasterblende aus einem transparenten Korpus (32) mit kreisringförmigen Blendenelementen (33) gebildet ist, die auftreffendes Licht aus einer Lichtquelle (25, 26) in einen Blendenraum (24) reflektieren, wobei die Beleuchtungseinrichtung zumindest eine erste Lichtquelle (25) aufweist, die Licht in einem monochromatischen Spektrum emittieren kann, wobei auf die Rasterblende Licht der ersten Lichtquelle auftrifft, derart, dass mittels der Rasterblende ein Bildmuster (35) mit monochromatischen Licht der ersten Lichtquelle auf einer Oberfläche eines Auges (13) abbildbar ist, wobei mittels der Beobachtungsvorrichtung Bilder des abgebildeten Bildmusters aufgenommen werden können, wobei aus den Bildern eine Topografie der Oberfläche abgeleitet werden kann, wobei die Beleuchtungseinrichtung zumindest eine weitere Lichtquelle (26) aufweist, die polychromatisches Licht in einem sichtbaren Spektrum zur Beleuchtung emittieren kann,
**dadurch gekennzeichnet,**
**dass** der Blendenraum gekrümmt ist und aus einem Reflektor (20) mit einer reflektierenden Fläche (22) und einer Oberfläche (23) des Reflektors gebildet ist, wobei die reflektierende Fläche und die Oberfläche stets den gleichen Abstand (a) aufweisen, wobei die Projektionsvorrichtung, die als ein Hohlkugelsegment (18) ausgebildet ist, den Reflektor umfasst, wobei auf die Rasterblende Licht der weiteren Lichtquelle auftrifft, derart, dass mittels der Rasterblende ein Bildmuster (35) mit sichtbaren polychromatischen Licht der weiteren Lichtquelle auf der Oberfläche eines Auges (13) abbildbar ist, wobei mittels der Beobachtungsvorrichtung Bilder des abgebildeten Bildmusters aufgenommen werden können.

2. Analysegerät nach Anspruch 1,
**dadurch gekennzeichnet,**
**dass** die erste Lichtquelle (25) Licht in einem infraroten Spektrum emittieren kann.

3. Analysegerät nach Anspruch 1 oder 2,
**dadurch gekennzeichnet,**
**dass** die weitere Lichtquelle (26) weißes Licht emittieren kann.

4. Analysegerät nach einem der vorangehenden Ansprüche,
**dadurch gekennzeichnet,**
**dass** die weitere Lichtquelle (26) aus einer Vielzahl gleichmäßig verteilten Leuchtdioden (28) zusammengesetzt ist.

## Claims

1. An ophthalmological analysis instrument (10), in particular for measuring a topography of an eye's surface, having a projection apparatus (11) and a monitoring apparatus (12), said projection apparatus comprising at least one illumination device and a raster screen (19), said raster screen being made of a transparent body (32) having annular aperture elements (33) which reflect incoming light from a light source (25, 26) into an aperture space (24), said illumination apparatus comprising at least one first light source (25) which can emit light in a monochromatic spectrum, light from the first light source coming in onto the raster screen in such a manner that an image pattern (35) having monochromatic light of the first light source can be imaged on an eye's (13) surface by means of the raster screen, images of the imaged image patterns being able to be recorded by means of the monitoring apparatus, a topography of the surface being able to be derived from the images, said illumination device comprising at least one other light source (26) which can emit polychromatic light in a visible spectrum for illumination,
**characterized in that**
the aperture space is curved and is made of a reflector (20) having a reflective surface (22) and a surface (23) of the reflector, said reflective surface and said surface always having the same distance (a), said projection apparatus being realized as a hollow-sphere segment (18) and comprising the reflector, light from the other light source coming in onto the raster screen in such a manner that an image pattern (35) having visible polychromatic light of the other light source being able to be imaged on an eye's (13) surface by means of the raster screen, images of the imaged image pattern being able to be recorded by means of the monitoring apparatus.

2. The analysis device according to claim 1,
**characterized in that**
the first light source (25) can emit light in an infrared spectrum.

3. The analysis device according to claim 1 or 2,
**characterized in that**
the other light source (26) can emit white light.

4. The analysis device according to any one of the preceding claims,
**characterized in that**
the other light source (26) is made of a plurality of evenly distributed light-emitting diodes (28).

## Revendications

1. Appareil d'analyse ophtalmologique (10), notamment pour mesurer une topographie d'une surface d'un oeil, comportant un dispositif de projection (11) et un dispositif d'observation (12), le dispositif de projection comprenant au moins un moyen d'éclairage et un moyen de diaphragme (19), ledit moyen de diaphragme étant réalisé d'un corps transparent (32) ayant un élément d'aperture annulaire (33) qui réfléchissent la lumière atteinte d'une source lumineuse (25, 26) dans un espace d'aperture (24), ledit moyen d'éclairage comportant au moins une première source lumineuse (25) qui peut émettre de la lumière dans un spectre monochromatique, de la lumière de la première source lumineuse atteignant sur le moyen de diaphragme de telle manière qu'un motif d'images (35) ayant de la lumière monochromatique de la première source lumineuse peut être imagé sur une surface d'un oeil (13) par le moyen de diaphragme, des images du motif d'images imagé pouvant être prises par le dispositif d'observation et une topographie de la surface pouvant être dérivée à partir des images, ledit moyen d'éclairage comprenant au moins une outre source lumineuse (26) qui peut émettre de la lumière polychromatique dans un spectre visible pour l'éclairage
**caractérisé en ce que**
l'espace d'aperture est courbé et se compose d'un réflecteur (20) ayant une surface réfléchissante (22) et une surface (23) du réflecteur, ladite surface réfléchissante et ladite surface ayant toujours la même distance (a), ledit dispositif de projection étant réalisé en tant que segment de sphère creuse (18) et comprenant le réflecteur, de la lumière de l'outre source lumineuse atteignant sur le moyen de diaphragme de telle manière qu'un motif d'images (35) ayant de la lumière polychromatique visible de l'outre source lumineuse peut être imagé sur la surface d'un oeil (13) par le moyen de diaphragme, des images du motif d'images imagé pouvant être prises par le dispositif d'observation.

2. Appareil d'analyse selon la revendication 1,
**caractérisé en ce que**
la première source lumineuse (25) peut émettre de la lumière dans un spectre infrarouge.

3. Appareil d'analyse selon la revendication 1 ou 2,
**caractérisé en ce que**
l'outre source lumineuse (26) peut émettre de la lumière blanche.

4. Appareil d'analyse selon l'une quelconque des revendications précédentes
**caractérisé en ce que**
l'outre source lumineuse (26) se compose d'une pluralité de diodes électroluminescentes (28) distribuées uniformément.
